# EUROPEAN PATENT SPECIFICATION

(11) **EP 0 930 053 B1**
(45) Date of publication and mention of the grant of the patent: **07.04.2004**
(21) Application number: 98100591.1
(22) Date of filing: 15.01.1998
(51) Int. Cl.: A61F 15/00

(54) **Stacked adhesively attachable disposable absorbent articles**
Gestapelte, durch Verklebung befestigte absorbierende Wegwerfartikel
Articles absorbants jetables empilés munies de dispositifs d'attache par adhésif

(43) Date of publication of application: 21.07.1999
(73) Proprietor: Hirsch, Uwe Thomas, 64347 Griesheim (DE)
(72) Inventor: Hirsch, Uwe Thomas, 64347 Griesheim (DE)

(56) References cited:
- WO-A-94/16659
- WO-A-96/33683
- WO-A-97/16147
- US-A- 3 367 334
- US-A- 5 429 631
- US-A- 5 704 932

## Description

The present invention relates to stacks of disposable absorbent articles such as sanitary napkins, pantiliners, or sweat inserts. Such articles are adhesively attached with their garment facing surface to the garment worn by the user of such articles. In particular the present invention relates to stacks of disposable absorbent articles which stacks are provided by using the wearer facing surface of one article in the stack as the release/protection surface to which the adhesive on the garment facing surface of a following article is attached prior to use.

### Background of the invention

Stacks of adhesively joined articles are well-known. For example the "Post It" ™ stickers by the Minnesota Mining and Manufacturing Company of Minnesota are adhesively attached to each other. They are forming a stack of stickers having a first side and a second side with the second side having an adhesive on it. The stacks are formed by attaching the adhesive of a second side of one sticker to the first surface of a following sticker within the stack. In this context the stack formation does not only provide protection of the adhesive prior to its intended use but also allows the formation of a stable stack which can be handled by the user of such stickers in a convenient fashion.

For disposable absorbent articles a similar approach has been disclosed in EP-A-422 922 in which an individual bandage dispenser in disclosed which comprises a continuous band of bandages which are attached with one adhesively covered side to the reverse side of the following article. These articles are, however, for application to the skin of a wearer, i.e. the adhesive is on that side which is intended to contact the ultimate user of such articles. In addition this disclosure does not show a reduction of release liner since the major part of the adhesive area is still covered with release liner which in the context of band-aids also serves the function of protection of that area intended to contact the wound. The purpose of the attachment of one article to the next is to automatically transport a next article into a dispensing position when pulling an article out of the dispenser.

The disclosure of EP-A-700 675 intends to reduce the amount of release liner employed for disposable absorbent article such as sanitary napkins or pantiliners in accordance with the present invention. However, the reduction is achieved by using one release liner on both sides such that two disposable absorbent articles share one release liner. Also no stacks are formed in this context since the stacks according to the present invention have all articles directed with their garment facing surface or their wearer facing surface in the same direction. An additional problem associated with this disclosure is that the wearer facing surface of each article is exposed and that it will be contacted by the user of such articles when the first of the two articles is detached from the release liner.

Multi pad absorbent articles are known for improving absorbency, cleanliness and soiling protection. DE 4,322,550 discloses the use of a two layer construct sanitary napkin which is placed in the undergarment and provides the option to remove the upper layer in order to have a fresh appearance of the remaining lower layer. A similar stock of up to 5 napkins is disclosed in US 5,429,631. As another alternative "Booster" inserts to increase the capacity of an absorbent article e.g. during heavy flow days for sanitary napkins or for babies with exceptionally large volumes of liquid intake/excretion or during the night, are also known in the art.

US-5,460,624 discloses sanitary napkins which can be applied on top of the wearer facing surface of another sanitary napkin by removing part of their liquid impermeable garment facing surface and therefore allowing them to not only absorb liquids but let liquid pass through to the underlying sanitary napkin such that they can be used as absorbency boosters. However, no disclosure of stacks of disposable absorbent articles in accordance with the present invention is provided in this reference.

WO-A-9416659 discloses a system of continues napkins or panty liners rolled up which have to be detached along perforations when used.

WO 96/33683 discloses sanitary napkins for direct attachment to the skin of a user. In this context also stacks of such articles are considered where the wearer facing surface, which is liquid impermeable and typically provided by a liquid impermeable film functions as the release surface for the following article. Since the garment facing surface of articles according to this disclosure provide a barrier function and are not in contact with the skin of a wearer when used as intended this release surface can be provided with any common release agent regardless of its compatibility with the skin of a user. Also since the adhesive in this disclosure is intended for adhesion to the skin of a wearer any residue adhesive will not be a major concern for liquid permeability or comfort or safety for a wearer.

Commonly used sanitary napkins are provided with an adhesive on their garment facing surface in order to allow attachment of the disposable absorbent article to a garment of the wearer. In order to maintain this adhesive intact it must not be exposed to the environment prior to use of the absorbent article such that it is common to cover the adhesive with a protective release liner most frequently a release paper which is siliconised at least on one surface.

It is hence an objective of the present invention to provide disposable absorbent articles with an adhesive on their garment facing surface, however, with a significant reduction in the quantity of release liner material required in comparison to common disposable absorbent articles. It is an additional objective according to the present invention to provide stacks of absorbent articles in which the wearer facing surface is treated such that its functionality as liquid receiving surface and transmitting surface is unimpaired or even improved by the additional function of releasing and protecting the adhesive of the following article within a stack.

The reduction of release liners for a series of disposable absorbent articles such as pantiliners or sanitary napkins satisfies several desires. Amongst others it reduces the need to dispose of the release liner which in many cases is inconvenient. It also provides a reduction in raw material consumption which is generally considered an environmental benefit whilst of course also reducing raw material cost of the article as delivered. Separately, a reduction in process steps, i.e. a simplification for the production of disposable absorbent articles, is achieved with the present invention while it is still possible to use commonly known process steps.

Further benefits and objectives of the present invention will become more apparent when considered in the context of the drawings and the following description.

### Summary of the invention

The present invention relates to a stack of disposable absorbent articles according to the appended claims. The absorbent articles each have a garment facing surface and a wearer facing surface. On the garment facing surface is an adhesive which is protected prior to use by being releasably joined to the wearer facing surface of the following article in the stack. The stacks are build from at least 10 articles stacked on top of each other. The disposable absorbent articles are selected from articles which are attached to garments rather than from articles which are attached to the wearer and therefore have an adhesive on the wearer facing surface of the article or a portion thereof. Typical articles according to the present invention are sanitary napkins, pantiliners or sweat inserts such as underarm sweat pads or shirt collar inserts. In particular pantiliners can benefit from the present invention since they are intended to be used daily or at an even higher frequency.

Preferably, the articles are stacked in registry to each other. These articles usually have a longitudinal axis. In a preferred embodiment the adhesive on the garment facing surface has an extension along the longitudinal axis which is less than the maximum length of the articles along the longitudinal axis. More preferably, the adhesive on the garment facing surface of the articles has a smaller extension in any direction parallel or perpendicular to the longitudinal axis such that each article provides an adhesive free periphery which allows easy grabbing to remove the article from the remainder of the stack.

Preferably, the distance between the periphery of the disposable absorbent article and the area of adhesive coverage on the disposable absorbent article is at least 3 mm, preferably between 4 and 25 mm, in at least one location along the periphery.

In order to provide the last article (when considering consumption of the articles) in a stack also with a protected adhesive on its garment facing surface one release liner can be employed per stack. Thereby, the reduction in release liner can simply be calculated by one over the number of articles per stack. In a more preferred option the stack is placed in a package such as a card board box wherein the surface of the package opposite the last article in the stack is providing the required release function of a release paper and thereby no release liner is required per stack resulting in total elimination of release liner material per stack.

### Brief description of the drawings

While the specification concludes with claims particularly pointing out and distinctly claiming the present invention it is believed that the invention will be better understood from the following description in conjunction with the accompanying drawings.

Figure 1 is a perspective view of a stack of pantiliners similar to an embodiment of the present invention except for the number of articles in the stack.

Figure 2 is a perspective view of a stack of pantiliners according to the present invention in a dispensing package.

### Detailed description of the invention

The present invention relates to disposable absorbent articles which are put above each other into a stack. The disposable absorbent articles are such articles which have an adhesive on the surface which in use is pointing away from the wearer of the article and is attached by the adhesive during use to a garment. This surface is called garment facing surface of the article while the opposite side facing the wearer during use is called wearer facing surface of the article. The adhesive on the garment facing surface of the article is a pressure sensitive adhesive which attaches to a surface upon touching it. Therefore, the adhesive requires to be protected prior to use, i.e. while the article is not yet placed on and attached to a garment.

Conventionally this protection is provided by a release liner which is a separate strip of material covering at least the adhesive region so as to prevent premature adhesion to surfaces not desired to be adhered to. According to the present invention this protection is provided by the wearer facing surface of another article such that the articles are put into a stack, one on top each other and thereby do not require the conventional release liner.

In order to provide the protection function as well as the required easy delimination the wearer facing surface of the articles have to provide the same function as a release liner while at the same time providing the usual functions of a wearer facing surface of an absorbent article such as liquid permeability.

Essentially all conventional disposable absorbent articles having a wearer facing surface and a garment facing surface comprising an adhesive for attachment to a garment can benefit from the present invention. However, particularly preferred articles are sanitary napkins or pantiliners. In the following the usual construction and materials for the usual construction of such articles is described in the context of pantiliners or sanitary napkins. However, other articles such as underarm sweat pads, shirt collar inserts or head bands can also benefit by being providing according to the present invention.

Disposable absorbent articles according to the present invention exhibit absorbency for bodily fluids, the protection of the user's garments from soiling, and improved physical comfort to the user (cushoning). The disposable absorbent article is described below by reference to a sanitary napkin or panty liners. The terms "sanitary napkin" or "panty liner", as used herein, refers to an article which is worn by females adjacent to the pudendal region and which is intended to absorb and contain the various body fluids which are discharged from the body (e.g., vaginal discharges, menses, and/or urine) and which is intended to be discarded after a single use. The disposable absorbent article is preferably thin, more preferably between 1 and 5 mm thick and can either be substantially flat prior to use or in a preshaped form.

The terms "joined" or "affixed", as used herein, encompasses configurations whereby a first member is directly connected to a second member and configurations whereby a first member is indirectly connected to a second member by connecting the first member to intermediate members which in turn are connected to the second member.

In a preferred embodiment a panty liner napkin of the present invention comprises a liquid pervious topsheet, a liquid impervious backsheet joined to the topsheet, and an absorbent core intermediate the topsheet and the backsheet. The panty liner has two main surfaces, a body contacting or wearer facing surface which is provided by the topsheet and a garment facing or contacting surface, which is provided by the backsheet.

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred topsheets for use in the present invention are typically selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the wearer remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The wearer facing surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the wearer facing surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

The topsheet typically extends across the whole of the absorbent structure and outside the area coextensive with the absorbent structure.

When referring to the topsheet a multi layer structure or a mono layer structure is contemplated. The hybrid topsheet mentioned above is such a multi layer design but other multi layer topsheets such as primary and secondary topsheet designs are also considered.

Besides the above criteria for topsheets the topsheet also needs to provide the release function as indicated above. This release function can be provided by any conventional method well-known in the art for release liners. In particular the wearer facing surface of the topsheet can be siliconised or treated a surfactant (which may already be present in some of the film topsheet designs indicated above). It is, however, generally dependent on the formulation of the adhesive on the garment facing surface of the article which treatment is necessary for the wearer facing surface of the article to provide the release function.

Positioned in fluid communication with, and typically underlying the topsheet is the absorbent core. The absorbent core provides fluid storage and distribution function and can also comprise multiple layers. The core can comprise any usual absorbent material or combinations thereof. It preferably comprises absorbent gelling materials usually referred to as "hydrogel", "superabsorbent", hydrocolloid" materials in combination with suitable carriers.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared form polymerizable, unsaturated, acid-containing monomers, such as acrylic acid, which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of panty liners.

An embodiment of the core, particularly useful in the application of the present invention, comprises a double layer tissue laminate which can be formed by folding the tissue onto itself. These layers can be joined to each other. Absorbent gelling material or other optional material can be comprised between the layers.

The absorbent core can include optional components normally present in absorbent webs such as odor control agents, in particular suitable zeolites or silicas.

The backsheet primarily prevents the exudates absorbed and contained in the absorbent core from wetting articles that contact the absorbent product such as underpants, pants, pyjamas and undergarments. The backsheet is preferably impervious to liquids (e.g. menses and/or urine) and usually manufactured from a thin plastic film.

The backsheet typically extends across the whole of the absorbent core and can extend onto and form part of the topsheet by folding around the absorbent core. Thereby a topsheet configuration as disclosed in US 4,342,314, column 16, lines 47 - 62 can be achieved without the requirement to selectively aperture the topsheet.

Preferably, the backsheet also provides breathability to the absorbent article by being at least water vapour permeable, preferably air permeable. The backsheet can be a laminate material e.g. of a combination of microporous film and/or non-woven material, and/or apertured formed film. Breathability if desired can be limited to the periphery or the center of the backsheet or it can be across the whole backsheet.

The garment facing surface provided by the backsheet is provided with an adhesive layer. The adhesive layer is a panty fastening adhesive which has to be a pressure sensitive adhesive allowing the user of the articles according to the present invention to place the article in a garment, for example in an undergarment for panty liners or sanitary napkins, or attach it to that garment by use of the adhesive.

Any adhesive or glue used in the art for such purpose can be used herein, with pressure sensitive adhesives being those most commonly employed. Suitable adhesives are century A-305-IV manufactured by The Century Adhesive Corporation and Instant Lock 34-2823 manufactured by the national starge company, both USA.

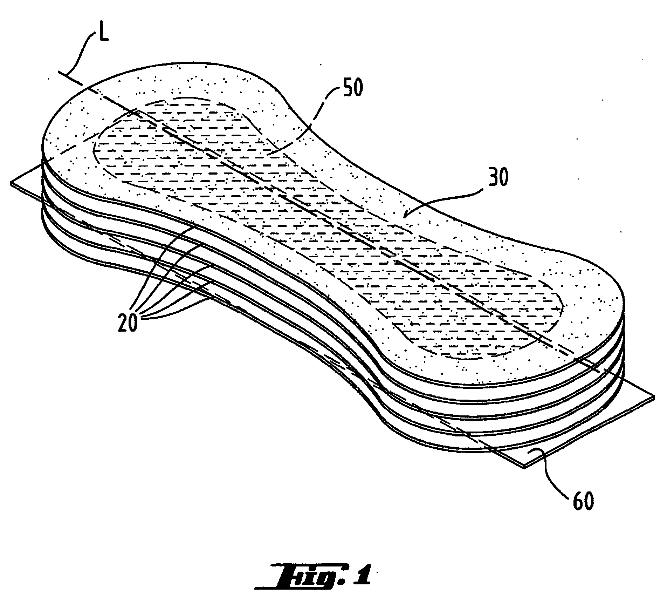

According to the present invention there is provided a stack of articles such as described above in which the adhesive on the garment facing surface of the article is joined to the wearer facing surface of the next article in order to form a stack. Referring to figure 1 a series of pantiliners, designated 20 can be seen. They are shown in a perspective view with the topsheet surface 30 visible. A panty fastening adhesive 50 is provided on the garment facing surface of the pantiliners 20. The adhesive 50 is coated on the garment facing surface within the area indicated by a dash line. The adhesive of the upper four panty liners is protected by the wearer facing surface of the lower most four panty liners 20 while the panty fastening adhesive on the garment facing surface of the lower most pantiliner 20 in figure 1 is protected by a single release paper 60 extending beyond the longitudinal length of the pantiliners. The pantiliners in figure 1 are stacked in registry to each other such that none extends in longitudinal direction or perpendicular thereto beyond any of the other pantiliners. The longitudinal direction is parallel to the longitudinal axis L indicated in figure 1 for the upper most pantiliner 20.

As can be seen in figure 1 the panty fastening adhesive 50 does not extend to the periphery of the pantiliners 20 and in fact follows the peripheral outline of the pantiliners 20 at a fixed distance.

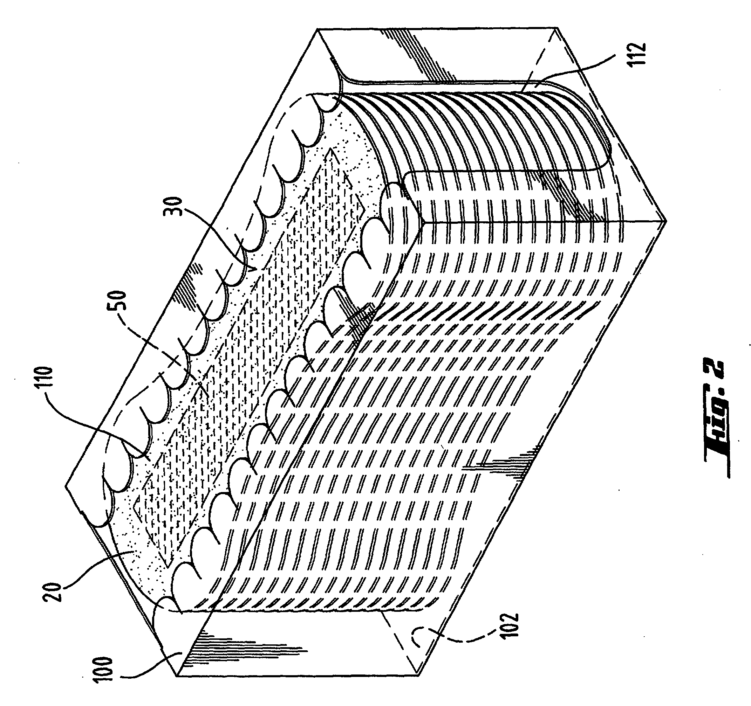

In figure 2 a similar stack, however, of more pantiliners 20 is shown in a carton package 100. The panty fastening adhesive 50 again is shown on the garment facing surface indicated by dashed hatching. The wearer facing surface 30 of the pantiliners 20 is visible in the opening 110 on top of the package. In order to allow easy access to the pantiliners 20 another opening 112 is provided on one side of the package such that a wearer who wants to use the pantiliner from the stack shown in figure 2 can easily grab the upper pantiliner and delaminate it from the wearer facing surface of the underlying pantiliner. The carton package 100 is made of such material that its bottom 102 provides a release surface for the lower most pantiliner 20 in the stack shown in figure 2 and thereby eliminates the need for any release paper for this stack. This can be provided by coating a release liner onto the bottom area 102 of the carton box at least in the area where the panty fastening adhesive of the lower most panty liner 20 will contact this part of the carton.

The articles according to the present invention can be prepared by a process for making such articles. The process starts with the provision of disposable absorbent articles. As described above such articles are usually prepared as layered structures and are as such made from a continuos band of the layers by putting the various materials making up the article onto each other and joining them to each other.

The topsheet and the backsheet of the articles provide the outer surfaces of the article and are directly or indirectly attached to each other around the periphery of the absorbent core. Topsheet and backsheet material are usually provided as roll goods in a continuos fashion thereby forming a continuos band or thread of the disposable absorbent articles prior to severing the articles from the thread.

According to the process this continuos thread of articles can be severed into individual articles followed by a step in which an adhesive for attaching the articles to garments is applied to the garment facing surface of the article. Alternatively these process steps can also be reversed such that the adhesive is applied to the garment facing surface of the articles while they are still in the format of a continuous thread.

After severing the articles from the continuous thread they are collected on top of each other such that the wearer facing surface of one article provides the release surface for the adhesive on the garment facing surface of the next article.

After severing the articles from the continuous thread of articles and after application of the adhesive the articles need to be conveyed to the unit where they are put into a stack. This can be achieved by holding the articles on said wearer facing surface on a conveyor belt by use of a suction force, for example by a vacuum conveyor belt well-known in the art.

This is particularly desirable if the articles are oriented such that the open adhesive surface is oriented downwards in a gravitational sense, i.e. such that the articles would fall down without being held in place on the conveyor belt. However, even if the articles or lying on said conveyor belt, i.e. with their garment facing surface oriented upwards in a gravitational sense it can be useful to hold them firmly on a vacuum conveyor belt by use of a suction force.

If the articles are oriented with the garment facing surface downwards they can be collected on top of each other by simply releasing them above a collection device which maintains their orientation and guides their fall onto the wearer facing surface of the previous articles. The first article to be collected in the collection device or receptacle needs to descend onto a release surface e.g. a release paper or if the collection device is the final container in which the stack of articles is to be transported and commercialized this container needs to have a release surface or coating on its bottom to allow release of the adhesive when consuming the last article from a stack.

It is preferable that the process further comprises the step of providing the wearer facing surface of the articles with a release substance. In particular it has been found that spraying of a release substance such as surfactants, silicon or teflon can achieve the desired result of providing easy and residue free release of the adhesive at the time of delaminating the article from the stack. Such release substances are particularly useful on apertured form film topsheets.

Alternative means of providing the release coating are for example roll coating, slot coating or printing. It will be apparent to those skilled in the art that the means of providing the release coating to the wearer facing surface will depend on many criteria, including sequence of steps, orientation of the articles, the article design and materials used for the article's wearer facing surface and conditions defined by the individual process environment.

While particular embodiments of the present invention have been illustrated and described those skilled in the art will recognize that various changes and modifications can be made. It is intended to cover in the appended claims .all such modifications that are part of the present invention.

## Claims

1. A stack of disposable absorbent articles (20), each article (20) having a garment facing surface, a wearer facing surface (30) and a garment facing adhesive (50) on said garment facing surface, said adhesive being protected prior to use, said articles having a longitudinal axis (L) and said stack being **characterized in that** said stack is formed by at least 10 individual articles (20) in a dispensing package (100), such that one article being releasable joined by said adhesive to said wearer facing surface of the following article in order to protect said adhesive prior to use.

2. A stack according to Claim 1 wherein said articles (20) are stacked in registry to each other.

3. A stack according to any of the preceding claims wherein said adhesive (50) has an outer periphery and an extension along said longitudinal axis (L), which is less than the maximum extension of said articles (20) along said longitudinal axis (L), preferably said outer periphery is spaced away everywhere from the periphery of said garment facing surface, more preferably said outer periphery is following the shape of said periphery of said garment facing surface, most preferably at a constant spacing of at least 3 mm, preferably between 4 and 25 mm.

4. A stack according to any of the preceding claims wherein said adhesive (50) on said garment facing surface of said article (20) forming the end on one side of said stack is covered by a release liner (60) surface.

## Revendications

1. Une pile d'articles absorbants à jeter (20), chaque article (20) ayant une surface montrer vers les vêtement , une surface montrer vers les utiliseur (30) et un adhésif (50) vers les vêtement sur la surface montrer vers les vêtement dite, l'adhésif dit étant protégé précédent pour utiliser, les articles dits ayant un axe longitudinal (L) et la pile dite étant caractérisée dans que la pile dite est formée par au moins 10 articles individuels (20) dans un paquet dispensant (100), tel qu'un article être libérable fixer par l'adhésif dit à surface montrer vers les utiliseur dit de l'article suivant afin de protéger l'adhésif dit précédent pour utiliser.

2. Une pile selon la revendication 1 dans lequel les articles dit(20) sont empilé dans l'enregistrement à chaque autre.

3. Une pile selon l'une quelconque des revendication précédentes dans lequel l'adhésif dit (50) a périphérie extérieur et une extension le long de l'axe longitudinal (L) dit, qui est moins que l'extension maximum d'articles dits (20) le long de l'axe longitudinal (L) dit, préférable la périphérie extérieure dite est loin espacée partout de la périphérie de surface montrer vers les vêtements dits, plus préférablement la périphérie extérieure dite suit la forme de périphérie de surface montrer vers la vêtement dite, le plus préférablement à une constante espaçant d'au moins 3 mm, préférablement entre 4 et 25 mm.

4. Une pile l'une quelconque des revendication précédentes dans lequel l'adhésif (50) sur la surface montrer vers les vêtement dite de les article (20) formant la fin sur un côté de pile dite est couverte par un paquebot de relâchement (60) la surface.

## Patentansprüche

1. Ein Stapel wegwerfbarer saugfähiger Artikel (20), jeder Artikel (20) umfasst eine zur Kleidung gerichtete Oberfläche, eine zum Träger gerichtete Oberfläche (30) und ein zur Kleidung gerichteten Klebstoff (50) auf der genannten zur Kleidung gerichteten Oberfläche, wobei der genannte Klebstoff (50) vor der Verwendung geschützt ist, die genannten Artikel beinhalten eine Längsachse (L), wobei der genannte Stapel **dadurch gekennzeichnet ist, dass** der genannte Stapel von wenigstens 10 einzelnen Artikeln (20) in einer Ausgabeverpackung (100) gebildet wird, und zwar so dass jeweils ein Artikel (20) durch den genannten Klebstoff auf der genannten zum Träger gerichteten Oberfläche (30) des folgenden Artikels (20) lösbar befestigt ist, um den genannten Klebstoff vor der Verwendung zu schützen.

2. Ein Stapel entsprechend Anspruch 1 wobei die genannten Artikel (20) deckungsgleich aufeinander gestapelt sind.

3. Ein Stapel entsprechend irgendeinem der vorhergehenden Ansprüche wobei der genannte Klebstoff (50) eine äußere Peripherie hat und eine Ausdehnung entlang der genannter Längsachse (L) hat, die weniger als die maximale Ausdehnung der genannten Artikel (20) entlang der genannten Längsachse (L) ist, vorzugsweise ist die genannte äußere Peripherie überall von der Peripherie der zur Kleidung gerichteten Oberfläche entfernt, vorzugsweise folgt die genannte äußere Peripherie des Klebstoffes der Peripherie der zur Kleidung gerichteten Oberfläche, am besten mit konstantem Abstand von wenigstens 3mm, vorzugsweise zwischen 4 und 25 mm

4. Ein Stapel entsprechend irgendeinem der vorhergehenden Ansprüche wobei der genannte Klebstoff (50) auf der zur Kleidung gerichteten Oberfläche der genannten Artikel (20) auf einer Endseite des genannten Stapels von einem Schutzfilm (60) abgedeckt wird.
